(19) Europäisches Patentamt
European Patent Office
Office européen des brevets

(11) EP 2 318 334 B1

(12) # EUROPEAN PATENT SPECIFICATION

(45) Date of publication and mention of the grant of the patent:
**17.07.2013 Bulletin 2013/29**

(21) Application number: **09788948.9**

(22) Date of filing: **20.07.2009**

(51) Int Cl.:
***C07C 1/207*** *(2006.01)* ***C07C 11/04*** *(2006.01)*

(86) International application number:
**PCT/US2009/004191**

(87) International publication number:
**WO 2010/014148 (04.02.2010 Gazette 2010/05)**

(54) **ETHYLENE PRODUCTION FROM ACETIC ACID UTILIZING DUAL REACTION ZONE PROCESS**

ETHYLENHERSTELLUNG AUS ESSIGSÄURE UNTER VERWENDUNG EINES VERFAHRENS MIT DUALER REAKTIONSZONE

PRODUCTION D´ÉTHYLÈNE À PARTIR D´ACIDE ACÉTIQUE PAR UN PROCESSUS À DOUBLE ZONE DE RÉACTION

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **31.07.2008 US 221138**

(43) Date of publication of application:
**11.05.2011 Bulletin 2011/19**

(73) Proprietor: **Celanese International Corporation**
**Dallas, TX 75234-6034 (US)**

(72) Inventors:
- **JOHNSTON, Victor, J.**
**Houston**
**TX 77059-3234 (US)**
- **ZINK, James, H.**
**League City**
**TX 77575 (US)**
- **CHAPMAN, Josefina, T.**
**Houston**
**TX 77059 (US)**
- **CHEN, Laiyuan**
**Houston**
**TX 77059 (US)**
- **KIMMICH, Barbara, F.**
**Bernardsville**
**NJ 07924 (US)**

(74) Representative: **Serravalle, Marco**
**Serravalle SAS**
**Via Matteotti, 21/23**
**26854 Cornegliano Laudense (LO) (IT)**

(56) References cited:
**JP-A- 2006 116 439**
**US-A- 4 398 039**
**US-A- 4 421 939**
**US-A- 4 480 115**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The present invention relates generally to a process for the production of ethylene from acetic acid. More specifically, the present invention relates to a process including hydrogenating acetic acid utilizing a first catalyst composition in a first reaction zone and dehydrating hydrogenated intermediates with a second catalyst in a second reaction zone to form ethylene with high selectivity.

### Background

There is a long felt need for an economically viable process to convert acetic acid to ethylene. Ethylene is an important commodity feedstock for a variety of industrial products; for example, ethylene can then be converted to a variety of polymer and monomer products. Fluctuating natural gas and crude oil prices contribute to fluctuations in the cost of conventionally produced, petroleum or natural gas-sourced ethylene, making the need for alternative sources of ethylene all the greater when oil prices rise.

It has been reported that ethylene can be produced from various ethyl esters in the gas phase in the temperature range of 150-300°C over zeolite catalysts. The types of ethyl esters that can be employed include ethyl esters of formic acid, acetic acid and propionic acid. *See,* for example, United States Patent No. 4,620,050 to Cognion et al.*,* where selectivity is reported to be acceptable.

US 4,421,939 discloses a three-step process for the preparation of ethylene from acetic acid, comprising: esterifying acetic add with an alcohol containing at least 4 carbon atoms; hydrogenating the ester to ethanol and a higher alcohol; dehydrating ethanol to ethylene.

United States Patent No. 4,270,015 to Knifton describes obtaining ethylene involving a two-step process in which a mixture of carbon monoxide and hydrogen (commonly known as synthesis gas) is reacted with a carboxylic acid containing 2 to 4 carbon atoms to form the corresponding ethyl ester of said carboxylic acid which is subsequently pyrolyzed in a quartz reactor at elevated temperatures in the range of about 200° to 600°C to obtain ethylene. The ethylene thus produced contains other hydrocarbons, particularly, ethane as an impurity. It was also reported therein that the concentration of ethane can reach high values, near 5% by pyrolyzing pure ethyl propionate at 460°C. More importantly, the conversion of the esters and yield of ethylene are reported to be very low.

United States Patent No. 4,399,305 to Schreck describes obtaining high purity ethylene from ethyl acetate employing a cracking catalyst composed of a perfluorosulfonic acid resin commercially sold under the trademark NAFION ® by E.I. DuPont de Nemours & Co.

On the other hand, Malinowski et al. Bull. Soc. Chim. Belg. (1985), 94(2), 93-5, disclose that reaction of substrates such as acetic acid on low-valent titanium heterogenized on support materials such as silica ($SiO_2$) or titania ($TiO_2$) resulted in a mixture of products including diethyl ether, ethylene and methane where selectivity is poor.

WO 2003/040037 discloses that crystalline microporous metalloaluminophosphates (ELAPO), particularly, SAPO-type zeolites, such as SAPO-5, SAPO-11, SAPO-20, SAPO-18 and SAPO-34, having Si/Al ratio of 0.03-017 are useful as adsorbent or as a catalyst for the production of olefins from an oxygenated feedstock containing methanol, ethanol, n-propanol, isopropanol, C4-C20 alcohols, methyl ethyl ether, di-methyl ether, di-ethyl ether, di-isopropyl ether, formaldehyde, dimethyl carbonate, dimethyl ketone and/or acetic acid. A similar disclosure utilizes a silicoaluminophosphate molecular sieves comprising at least one intergrown phase of molecular sieve. It is reported that in this process a feedstock containing an oxygenate contacts a catalyst comprising the molecular sieve in a reaction zone of a reactor at conditions effective to produce light olefins, particularly ethylene and propylene. *See* United States Patent No. 6,812,372 to Janssen et al. It is mentioned that such oxygenated feedstocks include acetic acid, but the disclosure appears to be limited to either methanol or dimethyl ether. *See, also,* United States Patent No. 6,509,290 to Vaughn et al.*,* which further discloses conversion of oxygenated feedstocks to olefins.

Bimetallic ruthenium-tin/silica catalysts have been prepared by reaction of tetrabutyl tin with ruthenium dioxide supported on silica. It has been reported that these catalysts exhibit different selectivities based on their content of tin/ruthenium ratio (Sn/Ru). Specifically it has been reported that the selectivity for the hydrogenolysis of ethyl acetate is quite different, which depends upon the Sn/Ru ratio in the catalyst. For instance, with ruthenium alone on $SiO_2$, the reaction is not selective: methane, ethane, carbon monoxide, carbon dioxide as well as ethanol and acetic acid are produced. Whereas, with low tin content, it has been reported that the catalysts are fairly selective for the formation of acetic acid, while at higher Sn/Ru ratios, ethanol is the only detected product. *See* Loessard et al., Studies in Surface Science and Catalysis (1989), Volume Date 1988, 48 (Struct. React. Surf.), 591-600.

The catalytic reduction of acetic acid has also been studied. For instance, Hindermann et al., J. Chem. Res., Synopses (1980), (11), 373, have disclosed the catalytic reduction of acetic acid on iron and on alkali-promoted iron. In their study they found that the reduction of acetic acid on alkali-promoted iron, followed at least two different routes

depending on the temperature. For example, they found that at 350°C, the Piria reaction was predominant and gave acetone and carbon dioxide, as well as they observed decomposition products methane and carbon dioxide, whereas the decomposition products were reduced at lower temperatures. On the other hand, at 300°C a normal reduction reaction was observed resulting in the formation of acetaldehyde and ethanol.

From the foregoing it is apparent that existing processes do not have the requisite selectivity to ethylene or existing art specifies starting material other than acetic acid, which are expensive and/or intended to produce products other than ethylene.

## Summary of the Invention

A process for selective formation of ethylene from acetic acid includes: contacting a feed stream containing acetic acid and hydrogen at an elevated temperature with a first catalytic composition including a suitable hydrogenating catalyst in a first reaction zone to form an intermediate mixture preferably including acetic acid, ethanol and ethyl acetate; and subsequently reacting said hydrogenated mixture over a suitable dehydrating and optionally a cracking catalyst in a second reaction zone to form ethylene.

## Brief Description of Drawing

The invention is described in detail bellow with reference to the single Figure which is a schematic diagram of a layered fix bed reactor.

## Detailed Description of the Invention

The invention is described in detail below with reference to numerous embodiments for purposes of exemplification and illustration only. Modifications to particular embodiments within the scope of the present invention, set forth in the appended claims, will be readily apparent to those of skill in the art.

Unless more specifically defined below, terminology as used herein is given its ordinary meaning % and like terms refer to mole percent unless otherwise indicated.

"Conversion" is expressed as a mole percentage based on acetic acid in the feed.

"Selectivity" is expressed as a mole percent based on converted acetic acid. For example, if the conversion is 50 mole % and 50 mole % of the converted acetic acid is converted to ethylene, we refer to the ethylene selectivity as 50%. Ethylene selectivity is calculated from gas chromatography (GC) data as follows:

$$\text{Ethylene Selectivity, \%} = 100^{*}\frac{mmol\ Ethylene\ out\ (GC)}{\frac{Total\ mmol\ C\ out\ (GC)}{2} - mmol\ AcOH\ out\ (GC)}$$

Without intending to be bound by theory, it is believed the conversion of acetic acid to ethylene in accordance with the invention proceeds in accordance with one or more of the following chemical equations:

### Step 1a: Hydrogenation of Acetic Acid to Ethanol.

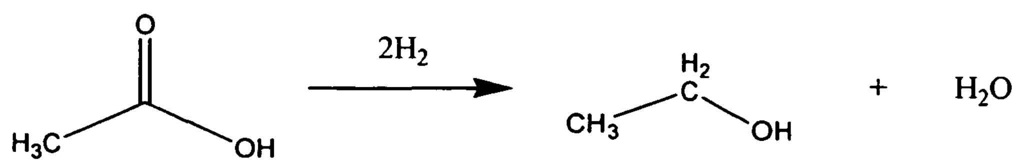

Step 1b: Hydrogenation of Acetic Acid to Ethyl Acetate

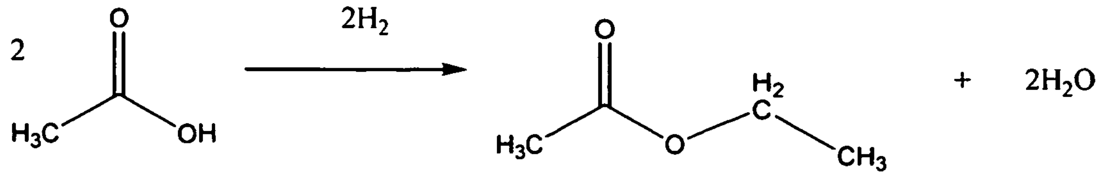


Step 1c: Cracking of Ethyl Acetate to Ethylene and Acetic Acid

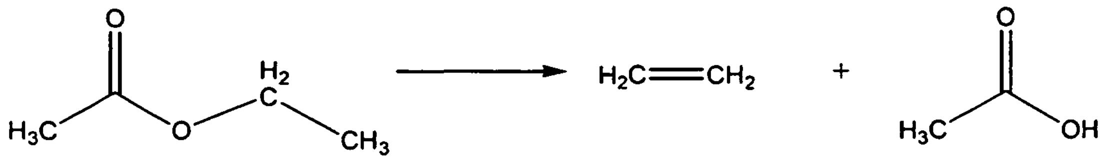

Step 2a: Dehydration of Ethanol to Ethylene.

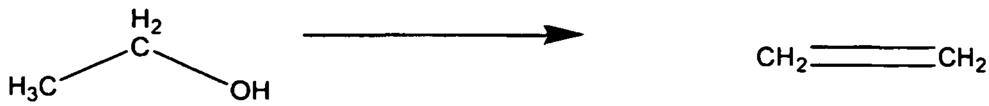

The process of the invention may be practiced in a variety of configurations using a fixed bed reactor or a fluidized bed reactor as one of skill in the art will readily appreciate. An adiabatic reactor could be used, or a shell and tube reactor provided with a heat transfer medium could be used. In any case, the two reaction zones may be housed in a single vessel with different layers in a fixed bed reactor or the two reaction zones may be housed in single vessel fluidized bed system with baffles and separators providing two distinct zones. Alternatively, two vessels could be used to house the different reaction zones. In any case, multiple reactors having two zones may be run in a parallel, for example, multiple tubular reactors having layered fixed beds arranged in parallel may be used if convenient.

There is shown schematically in **Figure 1** a tubular reactor with a layered fixed bed **10.** Bed **10** is a fixed bed in a vessel **12** which includes a layer of inert particulate material making up a mixing zone or layer **14,** a first reaction zone or layer **16,** an optional separator zone or layer **18,** a second reaction zone or layer **20** and a spacer zone or layer **22.** A reaction mixture including acetic acid, hydrogen and optionally an inert carrier gas is fed to bed **10** as a stream **24** under pressure to mixing zone **14.** The stream is subsequently supplied (by way of pressure drop) to first reaction zone or layer **16.** Reaction zone **16** comprises a first catalytic composition including a suitable hydrogenating catalyst where hydrogenated acetic acid intermediates are produced. Suitably, the first catalytic composition is in particulate form.

After hydrogenation, the mixture moves forward through optional separator zone **18** to second reaction zone or layer **20** containing a second catalytic composition comprising a suitable dehydration and/or cracking catalyst.

In zone **20,** the hydrogenated acetic acid intermediates such as ethyl acetate and ethanol are dehydrated and/or cracked to produce ethylene and the product is forwarded to spacer zone **22,** ultimately exiting bed **10** as product stream 26 at a pressure less than the inlet pressure to vessel **12.**

Layers **14, 18** and **22** are optional and may be formed of inert particulate material of suitable size in the configuration shown in **Figure 1.** In other layouts or configurations, equivalent means may be of any suitable design effective to promote mixing, separation, heat transfer and so forth as will be appreciated by one of skill in the art.

Various hydrogenation catalysts known to one skilled in the art can be employed in hydrogenating acetic acid to ethanol in the first step of the process of this invention. The hydrogenation catalysts that are suitable are the ones which are metal catalysts on a suitable support. Such catalysts are selected from the group consisting of : copper, nickel, aluminum, chromium, zinc, palladium a mixture thereof. Typically, either a single metal or a bimetallic catalyst on a suitable support can be used as a hydrogenation catalyst. Thus either copper alone or in combination with aluminum, chromium or zinc are particularly preferred.

Various catalyst supports known in the art can be used to support the catalysts of this invention. Examples of

such supports include without any limitation, iron oxide, silica, alumina, titania, zirconia, magnesium oxide, calcium silicate, carbon, graphite and a mixture thereof.

In an embodiment of this invention, specific examples of supported hydrogenation catalysts include iron oxide, silica, alumina, titania, zirconia, magnesium oxide, calcium silicate, carbon, graphite and a mixture thereof. Particularly, as noted above, copper supported on iron oxide and copper-aluminum catalysts are preferred.

A few of the commercially available catalysts include the following: copper-aluminum catalyst sold under the name of T-4489 by Sud Chemie; copper-zinc catalysts sold under the name of T-2130, T-4427 and T-4492; copper-chromium catalysts sold under the name of T-4419 and G-99B; and nickel catalysts sold under the name of NiSAT 310, C47-7-04, G-49, and G-69; all sold by Sud Chemie. Copper-aluminum catalyst sold under the name of T-4489 is particularly preferred.

The amount of metal loading on a support is not very critical in this invention and can vary in the range of about 3 weight percent to about 10 weight percent. A metal loading of about 4 weight percent to about 6 weight percent based on the weight of the support is particularly preferred. Thus, for example 4 to 6 weight percent of copper supported on iron oxide is particularly a preferred catalyst.

The metal impregnation can be carried out using any of the known methods in the art. Typically, before impregnation the supports are dried at 120°C and shaped to particles having size distribution in the range of about 0.2 to 0.4 mm. Optionally the supports may be pressed, crushed and sieved to a desired size distribution. Any of the known methods to shape the support materials into desired size distribution can be employed.

For supports having low surface area, such as for example alpha-alumina or iron oxide, the metal solutions are added in excess until complete wetness or excess liquid impregnation so as to obtain desirable metal loadings.

As noted above, a few of the hydrogenation catalysts are bimetallic. Generally, in such cases, one metal acts as a promoter metal and the other metal is the main metal. For instance copper, nickel,or cobalt are considered to be main metals for preparing hydrogenation catalysts of this invention. The main metal can be combined with a promoter metal such as chromium or zinc. However, it should be noted that sometimes main metal can also act as a promoter metal or vice versa. For example, nickel can be used as a promoter metal when iron is used as a main metal. Similarly, chromium can be used as a main metal in conjunction with copper (i.e., Cu-Cr as main bimetallic metals), which can further be combined with promoter metals such as zinc.

The bimetallic catalysts are generally impregnated in two steps. First, the "promoter" metal is added, followed by "main" metal. Each impregnation step is followed by drying and calcination. The bimetallic catalysts may also be prepared by co-impregnation. In the case of trimetallic Cu/Cr-containing catalysts as described above, a sequential impregnation may be used, starting with the addition of the "promoter" metal. The second impregnation step may involve co-impregnation of the two principal metals, i.e., Cu and Cr. Again, each impregnation is followed by drying and calcinations. In most cases, the impregnation may be carried out using metal nitrate solutions. However, various other soluble salts which upon calcination releases metal ions can also be used. Examples of other suitable metal salts for impregnation include metal hydroxide, metal oxide, metal acetate, ammonium metal oxide, such as ammonium heptamolybdate hexahydrate, metal acids, such as perrhenic acid solution or metal oxalate

A zeolite catalyst is employed as a dehydration catalyst. While any zeolite having a pore diameter of at least about 0.6 nm can be used, preferably employed among such zeolites are the dehydration catalyst selected from the group consisting of mordenites, ZSM-5, a zeolite X and a zeolite Y.

The preparation of large-pore mordenites is described, for example, in U.S. Pat. No. 4,018,514 and in Mol. Sieves Pap. Conf., 1967, 78, Soc. Chem. Ind. London, by D. DOMINE and J. QUOBEX.

Zeolite X is described, for example, in U.S. Pat. No. 2.882,244 and zeolite Y in U.S. Pat. No. 3,130,007.

Various zeolites and zeolite-type materials are known in the art for the catalysis of chemical reactions. For example, U.S. Pat. No. 3,702,886, *of Argauer,* discloses a class of synthetic zeolites, characterized as "Zeolite ZSM-5", which are effective for the catalysis of various hydrocarbon conversion processes.

The zeolites suitable for the procedure of the invention can be in the basic form, in the partially or totally acidified form, or in the partially dealuminated form.

The active catalyst in the process of the present invention, characterized as "H-ZSM-5" or "H-mordenite" zeolites are prepared from a corresponding "ZSM-5" zeolite or "mordenite" zeolite by replacing most, and generally at least about 80% of the cations of the latter zeolite with hydrogen ions using techniques well-known in the art. These zeolite catalysts are essentially crystalline aluminosilicates or in the neutral form a combination of silica and alumina in a well defined crystalline structure. In a particularly preferred class of zeolite catalysts for purposes of the present invention, the molar ratio of $SiO_2$ to $Al_2O_3$ in these zeolites is within the ratio of about 10 to 60.

As noted earlier, ethylene is produced by dehydration as well as the decomposition or "cracking" of ethyl acetate to ethylene and acetic acid. This may be a catalyzed reaction if so desired, utilizing a cracking catalyst. Suitable cracking catalysts include sulfonic acid resins such as perfluorosulfonic acid resins disclosed in United States Patent No. 4,399,305. noted above Zeolites are also suitable as cracking catalysts as noted in United States Patent No. 4,620,050. Thus, a zeolite catalyst may be used to concurrently dehydrate ethanol to ethylene and decompose ethyl acetate to ethylene in

a highly efficient process of the invention.

Selectivities of acetic acid to ethylene are suitably more than 10%, such as at least 20%, at least 40%, at least 60% or at least 80%. Depending on the by-product mix, it may be desirable to operate at intermediate selectivities, provided selectivity to undesirable products such as $CO_2$ remains low.

Preferably, for the purposes of the process of this invention, the suitable hydrogenation catalyst is either copper on iron oxide or copper-aluminum catalyst, sold under the tradename of T-4489 by Sud Chemie and the dehydration catalyst is H-mordenite. In this embodiment of the process of this invention, the copper loading on the iron oxide support or in the bimetallic copper-aluminum catalyst is typically in the range of 3 weight percent to 10 weight percent, preferably it is in the range of 4 weight percent to 6 weight percent.

In one of the embodiment of this invention, it is preferred that the hydrogenation and dehydration catalyst are layered. Preferably, the top layer of the catalyst bed is a hydrogenation catalyst and the bottom layer is dehydration catalyst.

In another aspect of the process of this invention, the hydrogenation and dehydration are carried out at a pressure just sufficient to overcome the pressure drop across the catalytic bed.

The reaction is carried out in the vapor phase. Reaction temperatures may be employed, for example in the range of 200°C to about 375°C, preferably 250°C to about 350° C. The pressure is generally uncritical to the reaction and subatmospheric, atmospheric or superatmospheric pressures may be employed. In most cases, however, the pressure of the reaction will be in the range of to 30 atmospheres absolute.

Although the reaction consumes two moles of hydrogen per mole of acetic acid to produce a mole of ethanol, the actual molar ratio of acetic acid to hydrogen in the feed stream may be varied between wide limits, e.g. from 100:1 to 1:100. It is preferred however that such ratio be in the range of about 1:20 to 1:2.

The raw materials used in connection with the process of this invention may be derived from any suitable source including natural gas, petroleum, coal, biomass and so forth. It is well known to produce acetic acid through methanol carbonylation, acetaldehyde oxidation, ethylene oxidation, oxidative fermentation, and anaerobic fermentation and so forth. As petroleum and natural gas have become more expensive, methods for producing acetic acid and intermediates such as methanol and carbon monoxide from alternate carbon sources have drawn more interest. Of particular interest is the production of acetic acid from synthesis gas (syngas) that may be derived from any suitable carbon source. United States Patent No. 6,232,352 to Vidalin, for example, teaches a method of retrofitting a methanol plant for the manufacture of acetic acid. By retrofitting a methanol plant the large capital costs associated with CO generation for a new acetic acid plant are significantly reduced or largely eliminated. All or part of the syngas is diverted from the methanol synthesis loop and supplied to a separator unit to recover CO and hydrogen, which are then used to produce acetic acid. In addition to acetic acid, the process can also be used to make hydrogen which is utilized in connection with this invention.

United States Patent No. RE 35,377 Steinberg et al., provides a method for the production of methanol by conversion of carbonaceous materials such as oil, coal, natural gas and biomass materials. The process include hydrogasification of solid and/or liquid carbonaceous material to obtain a process gas which is steam pyrolized with additional natural gas to form synthesis gas. The syngas is converted to methanol which may be carbonylated to acetic acid. The method likewise produces hydrogen which may be used in connection with this invention as noted above. *See also,* United States Patent No. 5,821,111 Grady et al., which discloses a process for converting waste biomass through gasification into synthesis gas as well as United States Patent No. 6,685,754 Kindig et al.

The acetic acid may be vaporized at the reaction temperature, and then it can be fed along with hydrogen in undiluted state or diluted with a relatively inert carrier gas, such as nitrogen, argon, helium or carbon dioxide.

Alternatively, acetic acid in vapor form may be taken directly as crude product from the flash vessel of a methanol carbonylation unit of the class described in United States Patent No. 6,657,078 of Scales et al. The crude vapor product may be fed directly to the reaction zones of the present invention without the need for condensing the acetic acid and light ends or removing water, saving overall processing costs.

Contact or residence time can also vary widely, depending upon such variables as amount of acetic acid, catalyst, reactor, temperature and pressure. Typical contact times range from a fraction of a second to more than several hours when a catalyst system other than a fixed bed is used, with preferred contact times, at least for vapor phase reactions, between 0.5 and 100 seconds.

Typically, the catalyst is employed in a fixed bed reactor e.g. in the shape of an elongated pipe or tube where the reactants, typically in the vapor form, are passed over or through the catalyst. Other reactors, such as fluid or ebullient bed reactors, can be employed, if desired. In some instances, it is advantageous to use the hydrogenation and zeolite catalysts in conjunction with an inert material such as glass wool to regulate the pressure drop of the reactant stream through the catalyst bed and the contact time of the reactant compounds with the catalyst particles.

In one of the preferred embodiments there is also provided a process for selective formation of ethylene from acetic acid comprising: contacting a feed stream of acetic acid and hydrogen in the vapor phase at a temperature in the range of 250°C to 350°C with a hydrogenation catalyst chosen from copper supported on iron oxide or copper-aluminum catalyst to form an intermediate mixture including acetic acid, ethanol and ethyl acetate; and concurrently reacting said

mixture over a dehydrating catalyst chosen from H-mordenite zeolite or sodium Y zeolite to form ethylene.

In this embodiment of the process of this invention, the preferred hydrogenation catalyst is 5 weight percent copper on iron oxide or 5 weight percent copper in a copper-aluminum catalyst and the dehydration catalyst is H-mordenite. In this embodiment of the process of this invention it is preferred that the hydrogenation and dehydration catalysts are layered in a fixed bed and the reaction is carried out in the vapor phase and at a temperature in the range of 300°C to 350°C and at a pressure in the range of 1 to 30 atmospheres absolute, and the contact time of reactants is in the range of 0.5 and 100 seconds.

The following examples describe the procedures used for the preparation of various catalysts employed in the process of this invention.

<u>Example A</u>

Preparation of 5 weight percent copper on Iron Oxide

Powdered and meshed iron oxide (100 g) of uniform particle size distribution of about 0.2 mm was dried at 120°C in an oven under nitrogen atmosphere overnight and then cooled to room temperature. To this was added a solution of copper nitrate (17 g) in distilled water (100 ml). The resulting slurry was dried in an oven gradually heated to 110°C (>2 hours, 10°C/min.). The impregnated catalyst mixture was then calcined at 500°C (6 hours, 1°C/min).

<u>Example B</u>

Preparation of H-Mordenite zeolite

H-Mordenite zeolite was prepared by calcination of ammonium form Mordenite at 500-550°C for 4-8 hours. If the sodium form of Mordenite is used as a precursor, the sodium Mordenite is ion-exchanged to ammonium form prior to calcination.

Gas Chromatographic (GC) analysis of the Products

The analysis of the products was carried out by online GC. A three channel compact GC equipped with one flame ionization detector (FID) and 2 thermal conducting detectors (TCDs) was used to analyze the reactants and products. The front channel was equipped with an FID and a CP-Sil 5 (20 m) + WaxFFap (5 m) column and was used to quantify:

Acetaldehyde
Ethanol
Acetone
Methyl acetate
Vinyl acetate
Ethyl acetate
Acetic acid
Ethylene glycol diacetate
Ethylene glycol
Ethylidene diacetate
Paraldehyde

The middle channel was equipped with a TCD and Porabond Q column and was used to quantify:

$CO_2$
Ethylene
Ethane

The back channel was equipped with a TCD and Molsieve 5A column and was used to quantify:

Helium
Hydrogen
Nitrogen
Methane

Carbon monoxide

Prior to reactions, the retention time of the different components was determined by spiking with individual compounds and the GCs were calibrated either with a calibration gas of known composition or with liquid solutions of known compositions. This allowed the determination of the response factors for the various components.

Example 1

The catalysts utilized were a copper on iron oxide catalyst, T-4489 purchased from Sud Chemie and an H-mordenite zeolite prepared by replacing with hydrogen ions all but 500 ppm based on the weight of the zeolite of the sodium ions in a sodium aluminosilicate mordenite catalyst prepared in accordance with United States Patent No. 4,018,514 or equivalent in which the ratio of silica to alumina is preferably in the range of from about 15:1 to about 100:1. A suitable catalyst is CBV21A available from Zeolyst International, which has a silica to alumina ratio of about 20:1.

In a tubular reactor made of stainless steel, having an internal diameter of 30 mm and capable of being raised to a controlled temperature, there are arranged 30 ml of 5 weight percent copper on iron oxide catalyst as top layer and 20 ml of H-mordenite as a bottom layer. The length of the combined catalyst bed after charging was approximately about 70 mm.

A feed liquid was comprised essentially of acetic acid. The reaction feed liquid was evaporated and charged to the reactor along with hydrogen and helium as a carrier gas with an average combined gas hourly space velocity (GHSV) of 2500 $hr^{-1}$ at a temperature of 300°C and pressure of 0.69 MPa (100 psig). The feed stream contained a mole percent of acetic acid from about 6.1 % to about 7.3% and mole percent of hydrogen from about 54.3% to about 61.5%. The feed stream was supplied to the hydrogenation catalyst (top) layer first such that the stream with hydrogenated acetic acid intermediates then contacted the dehydration catalyst layer. A portion of the vapor effluent from the reactor was passed through a gas chromatograph for analysis of the contents of the effluents. The acetic acid conversion was 65% and ethylene selectivity was 85%. Selectivity to acetone was 3%, selectivity to ethyl acetate was 2% and selectivity to ethanol was 0.6%. Carbon dioxide was relatively low; the measured selectivity to $CO_2$ of the acetic acid converted was 4%.

Example 2

The catalysts utilized were 5 weight percent copper on iron oxide prepared in accordance with the procedure of Example A and an H-mordenite zeolite prepared by replacing with hydrogen ions all but 500 ppm based on the weight of the zeolite of the sodium ions in a sodium aluminosilicate mordenite catalyst as noted above in Example 1.

The procedure as set forth in Example 1 was substantially repeated with an average combined gas hourly space velocity (GHSV) of 2500 $hr^{-1}$ of the feed stream of vaporized acetic acid, hydrogen and helium at a temperature of 350°C and pressure of 0.69 MPa (100 psig). The resulting feed stream contained a mole percent of acetic acid of about 7.3% and mole percent of hydrogen of about 54.3%. A portion of the vapor effluent was passed through a gas chromatograph for analysis of the contents of the effluents. The acetic acid conversion was 8% and ethylene selectivity was 18%.

Generally speaking, selectivities to ethylene above 10% or so are highly desirable; it being appreciated that the other by-products such as ethanol or ethyl acetate can be re-cycled to the reactor along with unreacted acetic acid, while still other by-products can be re-processed or used for fuel value. Selectivities to $CO_2$ of less than 10% are desired, preferably less than 5%.

Comparative Examples 1 - 5

These examples illustrate the reaction of acetic acid and hydrogen over a variety of catalysts wherein either no ethylene was formed and/or very low levels of ethylene was detected.

In all of these examples the procedure as set forth in Example 1 was substantially followed with the exception of using different catalysts as listed in Table 1. As summarized in Table 1, in all of these comparative examples only one single layer of catalyst was used. The reaction temperature and selectivity to ethylene are also tabulated in Table 1.

Table 1

| Reactor Bed | Catalyst | Reactor Temperature (°C) | Mol% $H_2$ in Feed Stream | Mol% Acetic Acid in Feed Stream | Ethylene Selectivity |
|---|---|---|---|---|---|
| Single Layer | 0.5%-1% Pd on Carbon | 250 - 350°C | 54.2% | 7.3% | 0% |
| Single Layer | 1 % Ru on Carbon | 250 - 350°C | 36.8% | 7.3% | 0% |
| Single Layer | 2% Pt on $Fe_2O_3$ | 350°C | 34.3% - 76.5% | 4.4%-7.3% | 0% - 1% |
| Single Layer | 2.58% Pd/5.05% Mo on $SiO_2$ | 250 - 350°C | 36.8% | 7.3% | 0% - 0.5% |
| Single Layer | 4.79% Cu on SiO2 | 400°C | 35.2% | 7.5% | 0% - 2.25% |

In these examples various other products including acetaldehyde, ethanol, ethyl acetate, ethane, carbon monoxide, carbon dioxide, methane, isopropanol, acetone and water were detected.

Although the invention has been illustrated by certain of the preceding examples, it is not to be construed as being limited thereby; but rather, the invention encompasses the generic area as hereinbefore disclosed. Various modifications and embodiments can be made without departing from the scope thereof.

## Claims

1. A process for selective formation of ethylene from acetic acid comprising:

   contacting a feed stream containing acetic acid and hydrogen in the vapor phase at a temperature of 200° to 375°C with a first catalytic composition including a hydrogenating catalyst containing a metal selected from the group consisting of copper, nickel, aluminum, chromium, zinc, palladium or a mixture thereof on a support in a first reaction zone to form an intermediate hydrogenated mixture; and
   reacting said intermediate hydrogenated mixture over a second catalytic composition which includes a dehydrating catalyst comprising a zeolite catalyst selected from the group consisting of H-mordenite, ZSM-5, a zeolite X and a zeolite Y in a second reaction zone to form ethylene.

2. The method according to Claim 1, wherein the first and second reaction zones comprise respectively a first layer of the first catalytic composition and a second layer of the second catalytic composition in a fixed bed.

3. The method according to Claim 1, wherein the first and second reaction zones are in separate vessels.

4. The method according to Claim 1, wherein the selectivity to ethylene based on acetic acid consumed is at least 20 percent.

5. The method according to Claim 1, wherein the selectivity to ethylene based on acetic acid consumed is at least 40 percent.

6. The method according to Claim 1, wherein the selectivity to ethylene based on acetic acid consumed is at least 60 percent.

7. The process according to Claim 1, wherein the support is selected from the group consisting of iron oxide, silica, alumina, titania, zirconia, magnesium oxide, calcium silicate, carbon, graphite and a mixture thereof.

8. The process according to Claim 1, the zeolite has a silica to alumina ratio ($SiO_2$/$Al_2O_3$) in the range of 10 to 60.

9. The process according to Claim 1, wherein said intermediate hydrogenated mixture comprises ethanol and ethyl acetate.

**10.** The process according to Claim 1, wherein the hydrogenating catalyst is copper on iron oxide and the dehydrating catalyst is H-mordenite.

**11.** The process according to Claim 1, wherein the hydrogenating catalyst is copper-aluminum catalyst and the dehydration catalyst is H-mordenite.

**12.** The process according to Claim 1, wherein hydrogenation and conversion to ethylene are carried out at a temperature in the range of 250° to 350°C.

**13.** The process according to Claim 1, wherein the reactants consist of acetic acid and hydrogen with a molar ratio in the range of 100:1 to 1:100, and the pressure is in the range of 1 to 30 atmospheres absolute.

**14.** The process according to Claim 1, wherein the reactants consist of acetic acid and hydrogen with a molar ratio in the range of 1:20 to 1:2, and the temperature is in the range of 300°C to 350°C.

**15.** A process for selective formation of ethylene from acetic acid according to claim 1 comprising:

contacting a feed stream containing acetic acid and hydrogen in the vapor phase at a temperature of 250° to 350°C with a first catalytic composition including a hydrogenating catalyst selected from the group consisting of copper on an iron oxide support or copper-aluminum catalyst on a support in a first reaction zone to form an intermediate hydrogenated mixture comprising acetic acid, ethanol and ethyl acetate; and
concurrently reacting said intermediate hydrogenated mixture over a second catalytic composition which includes a dehydrating catalyst comprising a zeolite catalyst selected from the group consisting of H-mordenite, and sodium zeolite Y in a second reaction zone to form ethylene.

## Patentansprüche

**1.** Ein Verfahren zur selektiven Bildung von Ethylen aus Essigsäure enthaltend:

Kontaktieren eines Essigsäure und Wasserstoff in der Dampfphase enthaltenden Zufuhrstroms bei einer Temperatur von 200° bis 375°C mit einer ersten katalytischen Zusammensetzung umfassend einen Hydrierungskatalysator enthaltend ein Metall ausgewählt aus der Gruppe bestehend aus Kupfer, Nickel, Aluminium, Chrom, Zink, Palladium oder einer Mischung davon auf einem Träger in einer ersten Reaktionszone, um eine hydrierte Zwischenmischung zu bilden; und
Umsetzen besagter hydrierter Zwischenmischung über einer zweiten katalytischen Zusammensetzung, die einen Dehydrierungskatalysator enthaltend einen Zeolithkatalysator ausgewählt aus der Gruppe bestehend aus H-Mordenit, ZSM-5, einem Zeolith X und einem Zeolith Y umfasst, in einer zweiten Reaktionszone, um Ethylen zu bilden.

**2.** Die Methode gemäß Anspruch 1, worin die erste und zweite Reaktionszone eine erste Schicht der ersten katalytischen Zusammensetzung beziehungsweise eine zweite Schicht der zweiten katalytischen Zusammensetzung in einem Festbett enthalten.

**3.** Die Methode gemäß Anspruch 1, worin die erste und zweite Reaktionszone in getrennten Gefäßen sind.

**4.** Die Methode gemäß Anspruch 1, worin die Selektivität für Ethylen basierend auf verbrauchter Essigsäure mindestens 20 Prozent beträgt.

**5.** Die Methode gemäß Anspruch 1, worin die Selektivität für Ethylen basierend auf verbrauchter Essigsäure mindestens 40 Prozent beträgt.

**6.** Die Methode gemäß Anspruch 1, worin die Selektivität für Ethylen basierend auf verbrauchter Essigsäure mindestens 60 Prozent beträgt.

**7.** Das Verfahren gemäß Anspruch 1, worin der Träger ausgewählt ist aus der Gruppe bestehend aus Eisenoxid, Siliziumoxid, Aluminiumoxid, Titanoxid, Zirkoniumoxid, Magnesiumoxid, Calciumsilikat, Kohlenstoff, Graphit und einer Mischung davon.

**8.** Das Verfahren gemäß Anspruch 1, der Zeolith hat ein Verhältnis von Siliziumoxid zu Aluminiumoxid ($SiO_2/Al_2O_3$) im Bereich von 10 bis 60.

**9.** Das Verfahren gemäß Anspruch 1, worin besagte hydrierte Zwischenmischung Ethanol und Ethylacetat enthält.

**10.** Das Verfahren gemäß Anspruch 1, worin der Hydrierungskatalysator Kupfer auf Eisenoxid ist und der Dehydrierungskatalysator H-Mordenit ist.

**11.** Das Verfahren gemäß Anspruch 1, worin der Hydrierungskatalysator Kupfer-Aluminium-Katalysator ist und der Dehydrierungskatalysator H-Mordenit ist.

**12.** Das Verfahren gemäß Anspruch 1, worin Hydrierung und Umwandlung zu Ethylen bei einer Temperatur im Bereich von 250° bis 350°C durchgeführt werden.

**13.** Das Verfahren gemäß Anspruch 1, worin die Reaktanden aus Essigsäure und Wasserstoff mit einem molaren Verhältnis im Bereich von 100:1 bis 1:100 bestehen und der Druck im Bereich von 1 bis 30 absoluten Atmosphären liegt.

**14.** Das Verfahren gemäß Anspruch 1, worin die Reaktanden aus Essigsäure und Wasserstoff mit einem molaren Verhältnis von 1:20 bis 1:2 bestehen und die Temperatur im Bereich von 300°C bis 350°C liegt.

**15.** Ein Verfahren zur selektiven Bildung von Ethylen aus Essigsäure gemäß Anspruch 1 enthaltend:

Kontaktieren eines Essigsäure und Wasserstoff in der Dampfphase enthaltenden Zufuhrstroms bei einer Temperatur von 250° bis 350°C mit einer ersten katalytischen Zusammensetzung umfassend einen Hydrierungskatalysator ausgewählt aus der Gruppe bestehend aus Kupfer auf einem Eisenoxidträger oder Kupfer-Aluminium-Katalysator auf einem Träger in einer ersten Reaktionszone, um eine hydrierte Zwischenmischung enthaltend Essigsäure, Ethanol und Ethylacetat zu bilden; und

gleichzeitiges Umsetzen besagter hydrierter Zwischenmischung über einer zweiten katalytischen Zusammensetzung, die einen Dehydrierungskatalysator enthaltend einen Zeolithkatalysator ausgewählt aus der Gruppe bestehend aus H-Mordenit und Natriumzeolith Y umfasst, in einer zweiten Reaktionszone, um Ethylen zu bilden.

**Revendications**

**1.** Procédé de formation sélective de l'éthylène à partir d'acide acétique, comprenant :

- mettre en contact un courant d'alimentation contenant de l'acide acétique et de l'hydrogène en phase vapeur à une température de 200° à 375°C avec une première composition catalytique comprenant un catalyseur d'hydrogénation contenant un métal choisi dans le groupe consistant en le cuivre, le nickel, l'aluminium, le chrome, le zinc, le palladium ou un mélange de ceux-ci sur un support dans une première zone de réaction pour former un mélange hydrogéné intermédiaire ; et
- faire réagir ledit mélange hydrogéné intermédiaire sur une seconde composition catalytique qui comprend un catalyseur de déshydratation comprenant un catalyseur zéolite choisi dans le groupe consistant en H-mordénite, ZSM-5, une zéolite X et une zéolite Y dans une seconde zone de réaction pour former de l'éthylène.

**2.** Procédé selon la revendication 1, dans lequel les première et seconde zones de réaction comprennent respectivement une première couche de la première composition catalytique et une seconde couche de la seconde composition catalytique dans un lit fixe.

**3.** Procédé selon la revendication 1, dans lequel les première et seconde zones de réaction sont dans des récipients séparés.

**4.** Procédé selon la revendication 1, dans lequel la sélectivité vis-à-vis de l'éthylène sur la base de l'acide acétique consommé est d'au moins 20 pour cent.

**5.** Procédé selon la revendication 1, dans lequel la sélectivité vis-à-vis de l'éthylène sur la base de l'acide acétique consommé est d'au moins 40 pour cent.

**6.** Procédé selon la revendication 1, dans lequel la sélectivité vis-à-vis de l'éthylène sur la base de l'acide acétique consommé est d'au moins 60 pour cent.

**7.** Procédé selon la revendication 1, dans lequel le support est choisi dans le groupe consistant en l'oxyde de fer, la silice, l'alumine, l'oxyde de titane, la zircone, l'oxyde de magnésium, le silicate de calcium, le carbone, le graphite et un mélange de ceux-ci.

**8.** Procédé selon la revendication 1, dans lequel la zéolite a un rapport silice à alumine ($SiO_2/Al_2O_3$) se situant dans la plage de 10 à 60.

**9.** Procédé selon la revendication 1, dans lequel ledit mélange hydrogéné intermédiaire comprend l'éthanol et l'acétate d'éthyle.

**10.** Processus selon la revendication 1, dans lequel le catalyseur d'hydrogénation est le cuivre sur de l'oxyde de fer et le catalyseur de déshydratation est la - mordénite.

**11.** Procédé selon la revendication 1, dans lequel le catalyseur d'hydrogénation est un catalyseur cuivre-aluminium et le catalyseur de déshydratation est la H-mordénite.

**12.** Procédé selon la revendication 1, dans lequel l'hydrogénation et la conversion en éthylène sont réalisées à une température dans la plage de 250° à 350°C.

**13.** Procédé selon la revendication 1, dans lequel les réactifs consistent en l'acide acétique et l'hydrogène ayant un rapport molaire dans la plage de 100:1 à 1:100, et la pression est dans la plage de 1 à 30 atmosphères absolues.

**14.** Procédé selon la revendication 1, dans lequel les réactifs consistent en l'acide acétique et l'hydrogène ayant un rapport molaire dans la plage de 1:20 à 1:2, et la température est dans la plage de 300°C à 350°C.

**15.** Procédé de formation sélective de l'éthylène à partir d'acide acétique selon la revendication 1, comprenant :

- mettre en contact un courant d'alimentation contenant de l'acide acétique et de l'hydrogène en phase vapeur à une température de 250° à 350°C avec une première composition catalytique comprenant un catalyseur d'hydrogénation choisi dans le groupe consistant en du cuivre sur un support d'oxyde de fer ou un catalyseur cuivre-aluminium sur un support dans une première zone de réaction pour former un mélange hydrogéné intermédiaire comprenant de l'acide acétique, de l'éthanol et de l'acétate d'éthyle ; et
- faire réagir simultanément ledit mélange hydrogéné intermédiaire sur une seconde composition catalytique qui comprend un catalyseur de déshydratation comprenant un catalyseur zéolite choisi dans le groupe consistant en H-mordénite et zéolite de sodium Y dans une seconde zone de réaction pour former de l'éthylène.

FIG. 1

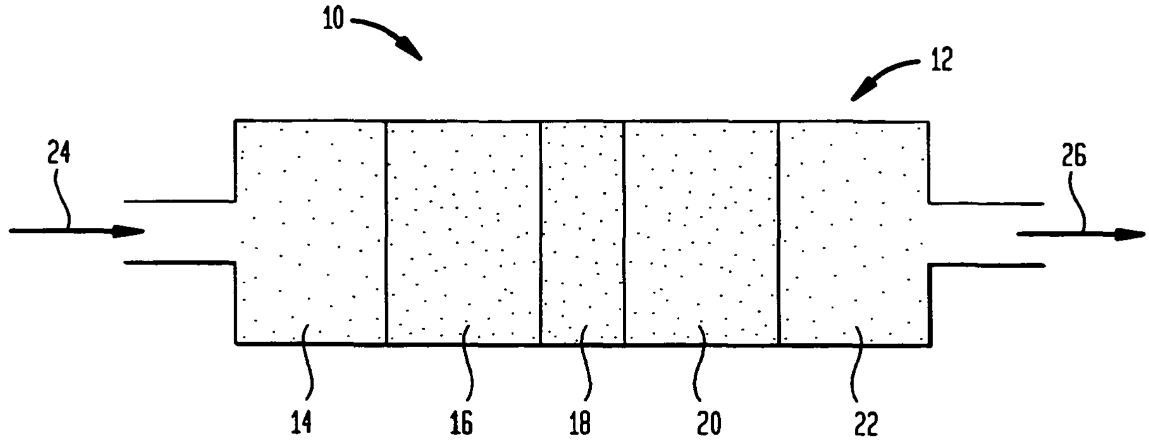

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4620050 A, Cognion
- US 4421939 A
- US 4270015 A, Knifton
- US 4399305 A, Schreck
- WO 2003040037 A
- US 6812372 B, Janssen
- US 6509290 B, Vaughn
- US 4018514 A
- US 2882244 A
- US 3130007 A
- US 3702886 A
- US 6232352 B, Vidalin
- US RE35377 E, Steinberg
- US 5821111 A, Grady
- US 6685754 B, Kindig
- US 6657078 B, Scales


### Non-patent literature cited in the description


- **MALINOWSKI et al.** *Bull. Soc. Chim. Belg.,* 1985, vol. 94 (2), 93-5
- **LOESSARD et al.** *Studies in Surface Science and Catalysis,* 1989, vol. 48, 591-600
- **HINDERMANN et al.** *J. Chem. Res., Synopses,* 1980, 373
- **D. DOMINE ; J. QUOBEX.** Mol. Sieves Pap. Conf. Soc. Chem. Ind, 1967, vol. 78